# EUROPEAN PATENT APPLICATION

(11) **EP 3 195 901 A1**
(43) Date of publication of application: **26.07.2017**
(21) Application number: 16152120.8
(22) Date of filing: 20.01.2016
(51) Int. Cl.: A61N 5/10

(54) **METHOD AND DEVICE FOR DETERMINING AN INTEREST OF APPLYING A QA PROCEDURE TO A TREATMENT PLAN IN RADIATION THERAPY**

(71) Applicant: ION BEAM APPLICATIONS S.A., 1348 Louvain-la-Neuve (BE)
(72) Inventor: DESSY, Frédéric, 1348 Louvain-la-Neuve (BE)
(74) Representative: Pecher, Nicolas

(57) **Abstract**

Computer-implemented method for determining an interest of applying a Quality Assessment (QA) procedure to a treatment plan in radiation therapy and comprising the steps of: (i) receiving information relating to said treatment plan; (ii) receiving information relating to a reference treatment plan that complies with a quality criterion; (iii) obtaining a dose distribution resulting from said reference treatment plan and/or a parameter of said reference treatment plan; (iv) obtaining a dose distribution resulting from said treatment plan and/or a parameter of said treatment plan; (v) comparing said dose distribution resulting from said treatment plan with said dose distribution resulting from said reference treatment plan, and/or said parameter of said treatment plan with same parameter of said reference treatment plan; (vi) from said comparison of step v, determining an interest of applying a QA procedure to said treatment plan.

## Description

### TECHNICAL FIELD

According to a first aspect, the invention relates to a computer-implemented method for determining an interest of applying a Quality Assessment (QA) procedure to a treatment plan in radiation therapy. According to a second aspect, the invention relates to a device for determining an interest of applying a QA procedure to a treatment plan in radiation therapy. According to a third aspect, the invention relates to a tangible machine readable storage medium. According to a fourth aspect, the invention relates to a program, for instance a computer program.

### DESCRIPTION OF RELATED ART

Nowadays, radiation therapy is widely used for treating tumors and cancers. As it is known by the one skilled in the art, different types of radiation beams can be used such as for instance beams of X-rays (or photons), electrons, protons, carbon ions or other ions. When protons or other ions are used, one skilled in the art generally use the terms of 'particle therapy'. Because of the type of radiation that is used, quality assessment (QA) of such treatments is of primary importance. QA procedures can be applied for instance to the radiation treatment system that is used (WO2007/038123 or WO2013/134597 for instance), or to a positioning system used in radiation therapy (US2014/0046601 for instance). Another important concern is to know or check the actual dose that is delivered and resulting from a given treatment plan, see for instance WO2009/114669. In particular, it is desired to deliver the correct dose to the regions to treat, while at a same time, not radiating healthy areas.

Before treating a patient, the following procedure is generally performed. From diagnostic data and from images of the patient, a practitioner (a doctor for instance) determines a dose distribution to deliver. Then, a treatment plan comprising various parameters related to the beamline that is used is determined (for instance by a medical physicist). When X-ray therapy is used, examples of such parameters are: a Multi Leaf Collimator (MLC) shape, a dose rate, beam energy, gantry angle, collimator angle. When particle therapy is used, examples of such parameters are: range, range modulation, shape of compensator, spot size, spot position, spot weight, spot tune, energy of an iso-energetic layer, Source to Axis Distance (SAD), range shifter, ridge filter. A treatment plan is generally determined by a Treatment Planning System (TPS) and/or by a machine design.

It is important that a dose distribution resulting from a treatment plan that is to be used is close to the dose distribution that is wanted and that has been determined by a doctor. Therefore, before treating the patient, a treatment plan often undergoes a Quality Assessment (QA) procedure.

As an illustrative example, a QA procedure for a treatment plan can comprise the following steps:
1. computing in a phantom, for instance with a TPS, a dose distribution induced by a radiation beam whose parameters are provided by said treatment plan;
2. measuring in an equivalent phantom (for instance with an ionization chamber) a dose distribution induced by a radiation beam whose parameters are provided by same treatment plan;
3. computing a difference between these computed and measured dose distributions;
4. if a magnitude of this difference is lower than a threshold, the treatment plan is recognized as being 'quality approved'; otherwise, the origin of this large difference is searched for modifying the treatment plan in order to finally have a 'quality approved' plan.
If a treatment plan is recognized as being 'quality approved', one can better expect that it will effectively provide or lead a dose distribution that is desired (within tolerated margins of errors).

In general, a QA procedure for a treatment plan is long and heavy to carry out. This is notably due to the following reasons: measuring a dose distribution in a phantom is long and computing a dose distribution induced by a radiation beam may also be long, especially in case of Monte Carlo calculations. For instance, the QA procedure of previous paragraph generally takes between 90 minutes and a full day, or even more. Step 1 alone (computation of dose distribution in a phantom) can take one day in some cases, e.g. in Monte Carlo calculations. Such long durations are problematic as they increase the costs notably. Therefore, it would be useful to know the interest of applying a QA procedure to a treatment plan.

The long durations associated to a QA procedure are also problematic in adaptive radiation therapy where it is desired that a treatment plan can be (preferably slightly) modified in real time during treatment for taking into account new data, such as a new position of the patient or a change of patient anatomy for instance. One major issue is then to perform a QA procedure of the new generated treatment plan. If such QA procedure is too long, on-line adaptive radiation therapy cannot be applied, as it is possible that new data for which the new treatment plan has been generated is no longer valid (after a QA procedure has been carried out). If the QA procedure is long, the duration and cost of adaptive radiation therapy also increase.

Document EP2116277 discloses a device and method for particle therapy monitoring and verification. A treatment beam comprises one or more treatment beam layers each characterized by treatment beam layer parameters. This Treatment Verification System (TVS) is used for monitoring a treatment in real time, during delivery of a treatment beam layer. The predicted 2D detector responses corresponding to each treatment beam layers are computed and stored in memory before performing the treatment. The treatment beam layers are then delivered and the corresponding 2D detector responses are measured in real time. If a difference between an expected value (the predicted 2D detector response) and actual values (the measured 2D detector response) is above a threshold, a signal is raised. This devices and method focuses on the verification that the delivered beam is of good quality, i.e. it is a Quality Control (QC) method and device, and is performed during or at the end of the treatment. This document does not address the question of Quality Assurance (QA) i.e. providing confidence before the treatment that quality requirements will be fulfilled in future treatments. More specifically, this document does not help the practitioner to decide whether he must perform a Quality Assurance (QA) procedure before delivering a treatment plan with a given radiation treatment apparatus.

Document WO2007059164 discloses a unified quality assurance (QA) for a radiation treatment delivery system. A target detector is positioned to a target position. A target image is taken and compared with a reference target image, for determining whether the actual target position coincides with the preset target position. The source is then moved to a preset source position. In the same way, it is determined whether the actual source position coincides with the preset target position. Finally, a preset dose is delivered and the actual dose delivered is compared with the expected dose. All these steps focus on the alignment and calibration of components of the radiation treatment delivery system. The issue of quality assurance of a treatment plan, adapted to the treatment of a patient, is not addressed in this document. As discussed in previous paragraph, this document is also silent about the need to perform a Quality Assurance of a treatment plan.

There is therefore a need to provide a method and/or a device that could help deciding whether a QA procedure needs or does not need to be applied to a treatment plan.

### SUMMARY OF THE INVENTION

According to a first aspect, the invention provides a computer-implemented method for determining an interest of applying a Quality Assessment (QA) procedure to a treatment plan in radiation therapy and comprising the steps of:
i receiving information relating to said treatment plan;
ii receiving information relating to a reference treatment plan that complies with a quality criterion,
iii obtaining a dose distribution resulting from said reference treatment plan and/or a parameter of said reference treatment plan;
iv obtaining a dose distribution resulting from said treatment plan and/or a parameter of said treatment plan;
v comparing:
   a. said dose distribution resulting from said treatment plan with said dose distribution resulting from said reference treatment plan, and/or
   b. said parameter of said treatment plan with same parameter of said reference treatment plan);
vi from said comparison of step v, determining an interest of applying a QA procedure to said treatment plan.

Said dose distribution resulting from said treatment plan and/or said parameter of said treatment plan may be obtained from a treatment planning system or obtained by a computation

Said dose distribution resulting from said reference treatment plan and/or said parameter of said reference treatment plan may be obtained from a treatment planning system or obtained by a computation.

The method is preferably performed prior to the delivery of said treatment plan.

Preferably, determining an interest has to be construed as determining a level of interest. For example, a two level of interest: no interest/interest, or a three level of interest: no interest/small interest/interest. In another example, a level of interest can be determined as a continuous value between for example 0 and 1. Preferably, the method according to the invention corresponds to a method to determine if a QA procedure needs to be applied to a treatment plan before this treatment plan can be used for radiation therapy. Preferably, the treatment plan has to be construed as a patient treatment plan.

Thanks to the method of the invention, one can have information on the interest of performing a QA procedure to a treatment plan. From this information, one can better evaluate the requirement of performing such a QA procedure. In particular, one can save time and money if there is no great interest or no interest at all in applying a QA procedure to a treatment plan. With the method of the invention, adaptive radiation therapy is easier to perform: by knowing that QA procedures do not need to be applied to some possible treatment plans (small interest determined in step vi for instance), one can directly apply these treatment plans without having to carry out QA procedures.

The method of the invention has other advantages. It is simple: indeed, one only needs to provide at least one reference treatment plan. It is also very quick an efficient. In step v, it is possible to compare only one or more parameters between said treatment plan and said reference treatment plan. Then, one does not need to determine dose distributions resulting from said treatment plans. This preferred version further increases the simplicity of the method of the invention.

As it is known by the one skilled in the art, examples of radiation therapy are: X-ray therapy (therapy using photons), and therapy using energetic ionizing particles (often named particle therapy) such as protons or carbon ions for instance. The method of the invention can be applied in Intensity-Modulated Radiation Therapy (IMRT), and examples of X-ray therapy as it is known by the one skilled in the art. The invention may be used in static IMRT beam delivery methods as well as dynamic beam delivery methods such as VMAT® (Elekta) and Rapidarc® (Varian). The method of the invention could be applied with different techniques of particle therapy such that scattering beam and pencil beam scanning for instance.

The reference treatment plan of step ii. is for instance a quality approved treatment plan. A quality approved treatment plan is a treatment plan that has passed a QA procedure, and that therefore complies with at least one quality criterion. An example of QA procedure has been detailed above, when discussing related prior art in the field. According to another embodiment, the reference treatment plan is not a quality approved treatment plan. It could be for instance a treatment plan that is recognized as being a reference treatment plan by a practitioner because it satisfies at least one quality criterion. For instance, it could be a treatment plan that is known to induce a desired dose in a phantom with acceptable margins or errors. A team of practitioners can have such a reference treatment plan, notably from previous treatments. A reference treatment plan can also be a treatment plan generated from class solution where, mainly, the number of beams, and beam orientations have been validated. A class solution is for instance a treatment plan for which a physicist for instance has determined that some parameters are correct for a beam delivery.

Concept of treatment plan in radiation therapy is known by the one skilled in the art. A treatment plan comprises various parameters related to the radiation setup that is used. A treatment plan is generally determined by a medical physicist, from the knowledge of the desired dose distribution that is imposed generally by a doctor. Information of step i and ii can comprise for instance various parameters of the treatment plan such as for instance Multi Leaf Collimator (MLC) shape, dose rate, beam energy, gantry angle, collimator angle, range, range modulation, shape of compensator, spot size, spot position, spot weight, spot tune, energy of an iso-energetic layer, Source to Axis Distance (SAD), range shifter, ridge filter). Information of step i and ii can also comprise for instance one or more dose distributions induced by the treatment plan and the reference treatment plan. Other examples of information relating to the treatment plan and to the reference treatment plan are possible.

A treatment plan can relate to one or several radiation beams. When a treatment needs or uses several radiation beams, the associated treatment plan generally comprises parameters relating to these several radiation beams. Information relating to the treatment plan and to the reference treatment plan in steps i and ii can relate to one or several radiation beams. In particular, when the treatment plan and the reference treatment plan relate to several radiation beams, steps i and ii of the method of the invention can consist in providing information relating to only one beam of said treatment plan and said reference treatment plan. This is notably preferred when step v consists in comparing a dose distribution (and/or a parameter) resulting from (of) only one beam. However, when several radiation beams are used for treatment, and when the treatment plan and the reference treatment plan relate to several beams, one could perform the comparison of step v for several beams. Either by performing comparison for each beam individually or by performing a global comparison for the different beams of the plans together. In the latter case, one could indeed compare a global dose distribution and/or a global parameter relating to the different beams together.

Preferably, step i is 'providing said treatment plan'. Preferably, step ii is 'providing a reference treatment plan that complies with a quality criterion'.

Word 'parameter' in step v. could be replaced for instance by setting parameter, by experimental output, or by setting. Words 'resulting from' in step v. could be replaced by 'induced by'. Preferably, the dose distributions of step v resulting from the treatment plan and from the reference treatment plan are computed dose distributions in a phantom. These dose distributions are preferably induced by the radiation beam(s) of the treatment plan and of the reference treatment plan. According to a possible embodiment, a plurality of dose distributions is compared in step v between the treatment plan and the reference treatment plan.

In step v, a plurality of parameters could be compared between the treatment plan and the reference treatment plan.

For performing the comparison of step v, mathematical operations between dose distributions and/or parameters of said treatment plan and of said reference treatment plan can be performed for instance. Examples of such mathematical operations are: difference, convolution, integration of differences on a surface. In step v, the dose distribution can result from one beam or from several beams of said treatment plan (respectively of said reference treatment plan).

Preferably, the method of the invention is a computer-implemented method.

Preferably, said comparison in step v comprises a step of determining a deviation Δ. As this comparison is performed between dose distribution(s) and/or parameter(s) of said treatment plan and said reference treatment plan, this deviation Δ can be named deviation Δ between said treatment plan and said reference treatment plan. Preferably, in step vi, the interest of applying a QA procedure to said treatment plan is then determined from said deviation Δ, and more preferably from a magnitude of said deviation Δ. More than one deviation Δ could be determined in this preferred embodiment.

Preferably, said step vi comprises a step of determining that a QA procedure should be applied to said treatment plan if magnitude of said deviation Δ is larger than a threshold, Δ₂.
Threshold Δ₂ is for instance determined by a practitioner. A practitioner can be a medical physicist for instance. This preferred embodiment presents the advantage of determining particularly clearly if a QA procedure should be applied.

Preferably, said step vi comprises a step of determining that a QA procedure does not need to be applied to said treatment plan if magnitude of said deviation Δ is lower than a threshold, Δ₁.
This preferred embodiment presents the advantage of determining particularly clearly that a QA procedure does not need to be applied.
According to a possible embodiment, Δ1= Δ2. According to another preferred embodiment, Δ1 < Δ2.
When such two thresholds are used, such that Δ1 < Δ2, step v. preferably determine the following interests of applying a QA procedure to said treatment plan:
- Δ>Δ2: interest is large or strongly recommended;
- Δ1<Δ<Δ2: interest is average;
- Δ<Δ1: interest is low or zero.

Preferably, the method of the invention comprises a step of providing information of the interest of applying a QA procedure to said treatment plan in the form of a flag that can take different colors. Then, the visualization and understanding of said interest by a user is particularly simple and clear. For instance, three colors could be used: green, orange, and red. In such a case, the signification of these three colors could be the following. When the flag is green, it means that interest of applying a QA procedure to said treatment plan is low or zero (this corresponds for instance to a case where Δ < Δ1 as defined above). Preferably, a QA procedure is then not applied to the treatment plan. When the flag is orange, it means that a practitioner should give his input to the interest of applying a QA procedure to said treatment plan, for instance, depending on his experience (this corresponds for instance to a case where Δ1<Δ<Δ2 as defined above). When the flag is red, it means that it is strongly recommended and mandatory to apply a QA procedure to said treatment plan (this corresponds for instance to a case where Δ > Δ₂ as defined above). Preferably, a QA procedure is then applied to the treatment plan.

Preferably, said parameter that can be compared between said treatment plan and said reference treatment plan in step v is able to influence a fluence in radiation therapy.
The inventors have found that the method of the invention is particularly efficient when choosing parameters able to influence a fluence in step v.
Said fluence is fluence at nozzle exit for instance. Fluence is known by the one skilled in the art. It represents a number of particles (protons, carbon ions for instance) or photons per unit area. Examples of parameters able to influence a fluence in radiation therapy are given below.

For instance, said parameter that can be compared between said treatment plan and said reference treatment plan in step v is a beam energy.
This possible embodiment of the method of the invention is preferably used when radiation therapy uses photons (X-ray therapy).

For instance, said parameter that can be compared between said treatment plan and said reference treatment plan in step v is a fluence. This possible embodiment of the method of the invention is preferably used when radiation therapy uses photons (X-ray therapy).

For instance, said parameter that can be compared between said treatment plan and said reference treatment plan in step v is a spot size.

By using a spot size for the parameter in step v, one has a parameter that is linked to a number of particles per unit area. And the inventors have found that the method is particularly efficient when using a parameter in step v that is linked to a number of particles per unit area.

This possible embodiment of the method of the invention is preferably used when radiation therapy uses particles (whose examples are protons and carbon ions), and more preferably when radiation therapy uses a technique of pencil particle beam scanning.

Said parameter that can be compared between said treatment plan and said reference treatment plan in step v is for instance a spot position. By using a spot position for the parameter in step v, one has a parameter that is linked to a number of particles per unit area. And the inventors have found that the method is particularly efficient when using a parameter in step v that is linked to a number of particles per unit area.
This possible embodiment of the method of the invention is preferably used when radiation therapy uses particles (whose examples are protons and carbon ions), and more preferably when radiation therapy uses a technique of pencil particle beam scanning.

Said parameter that can be compared between said treatment plan and said reference treatment plan in step v is for instance a spot weight. By using a spot weight for the parameter in step v, one has a parameter that is linked to a number of particles per unit area. And the inventors have found that the method is particularly efficient when using a parameter in step v that is linked to a number of particles per unit area.

This possible embodiment of the method of the invention is preferably used when radiation therapy uses particles (whose examples are protons and carbon ions), and more preferably when radiation therapy uses a technique of pencil particle beam scanning.

Said parameter that can be compared between said treatment plan and said reference treatment plan in step v may for instance comprise a beam limiting device position. A beam limiting device may comprise a multi-leaf collimator or jaws, or a specific aperture.

Preferably, several parameters are compared in step v between said treatment plan and said reference treatment plan. Then, several variations are possible. According to first example, one deviation Δ is directly determined in step v from the comparison of several parameters, and said interest of applying a QA procedure to said treatment plan is determined from said single deviation Δ in step vi. According to another example, several deviations Δ are determined in step v from the comparison of several parameters. Preferably, one deviation Δ is determined for each parameter that is compared. From such several deviations Δ, it is possible to determine a global deviation Δ from which the interest of applying a QA procedure to said treatment plan is determined in step vi. But it is also possible to determine the interest of applying a QA procedure to said treatment plan in step vi from said several deviations Δ, without having previously determine a global deviation Δ.

Preferably, said step v comprises a step of comparing the following four parameters between said treatment plan and said reference treatment plan: energy, spot position, spot weight, and spot size. The inventors have found that comparing these four parameters is particularly useful for knowing the interest of applying a QA procedure to said treatment plan. This preferred embodiment of the method of the invention is preferably used when radiation therapy uses particles (whose examples are protons and carbon ions), and more preferably when radiation therapy uses a technique of pencil particle beam scanning. As explained in previous paragraph, several variations for performing the comparison of step v are then possible. Spot weight can be an absolute spot weight or a relative spot weight.

Preferably, said parameter that can be or that is compared between said treatment plan and said reference treatment plan in step v is a gantry angle.

Preferably, said parameter that can be or that is compared between said treatment plan and said reference treatment plan in step v is a dose rate. When this preferred embodiment of the method of the invention is followed, said radiation therapy is preferably X-ray therapy.

Preferably, said parameter that can be or that is compared between said treatment plan and said reference treatment plan in step v is a collimator angle. When this preferred embodiment of the method of the invention is followed, said radiation therapy is preferably X-ray therapy.

Preferably, said parameter that can be or that is compared between said treatment plan and said reference treatment plan in step v. is a spot tune ID. Spot tune ID is known by the one skilled in the art. It represents an identifier of a spot. It is related to spot size, and could be named 'spot size label'.

Preferably, said parameter that can be or that is compared between said treatment plan and said reference treatment plan in step v is an energy of an iso-energetic layer of a treatment plan.

This parameter generally varies with changes in patient anatomy. Its fluctuation therefore reflects changes in patient anatomy. By comparing this parameter in step v, one therefore has larger possibility of taking into account patient anatomy and its changes. This possible embodiment of the method of the invention is preferably used when radiation therapy uses particles (whose examples are protons and carbon ions), and more preferably when radiation therapy uses a technique of pencil particle beam scanning.

The method of the invention has no therapeutic effect. Therefore, it is not a therapeutic method. It allows knowing the interest of applying a QA procedure to a treatment plan.

According to a second aspect, the invention relates to a device for determining an interest of applying a Quality Assessment (QA) procedure to a treatment plan in radiation therapy and comprising:
- input means adapted for receiving:
   o information relating to said treatment plan,
   o information relating to a reference treatment plan that complies with a quality criterion,
- input means adapted for receiving and/or processing means adapted for computing:
   o a dose distribution resulting from said reference treatment plan and/or a parameter of said reference treatment plan;
   o a dose distribution resulting from said treatment plan and/or a parameter of said treatment plan;
- processing means adapted for:
   o comparing:
      ▪ said dose distribution resulting from said treatment plan with said dose distribution resulting from said reference treatment plan, and/or
      ▪ said parameter of said treatment plan with same parameter (21) of said reference treatment plan; and for
   o determining an interest of applying a QA procedure to said treatment plan from said comparison.

By using the device of the invention, one can have information on the interest of performing a QA procedure to a treatment plan. From this information, one can better evaluate the requirement of performing such a QA procedure. In particular, one can save time and money if there is no great interest or no interest at all in applying a QA procedure to a treatment plan. By using the device of the invention, adaptive radiation therapy is easier to perform: by knowing that QA procedures do not need to be applied to some possible treatment plans, one can directly apply these treatment plans without having to carry out QA procedures that are often long, and so not suitable for adaptive radiation therapy.

The device of the invention has other advantages. It can be a computer, or a port of a TPS, or a hardware module able to communicate with a TPS for instance. So, the device of the invention is simple. Only one or more reference treatment plans need to be provided to the device for it being able to determine an interest of applying a QA procedure to a treatment plan. In the step of comparison performed by the processing means, it is possible to compare only one or more parameters between said treatment plan and said reference treatment plan. Then, one does not need to determine dose distributions resulting from said treatment plans. This preferred version further increases the simplicity of the method performed by the device of the invention.

Different embodiments of the device of the invention are possible. In particular, the different embodiments of the method (first aspect of the invention) apply to the device, mutatis mutandis. The corresponding advantages of the different embodiments of the method of the invention apply to the device, mutatis mutandis.

Preferably, said processing means is able to determine a deviation Δ between said treatment plan and said reference treatment plan from said comparison.

Preferably, said determination of said interest of applying a QA procedure to said treatment plan comprises a step of determining that a QA procedure should be applied to said treatment plan if said deviation Δ is larger than a threshold, Δ₂.

Preferably, said determination of said interest of applying a QA procedure to said treatment plan comprises a step of determining that a QA procedure does not need to be applied to said treatment plan if said deviation Δ is lower than a threshold, Δ₁.

Preferably, the following four parameters are compared by said processing means between said treatment plan and said reference treatment plan: energy, spot position, spot weight, and spot size.

Preferably, the device of the invention is able to provide a message comprising the interest of applying a QA procedure to said treatment plan and that has been determined by said processing means. Preferably, the device of the invention comprises display means for displaying such a message.

According to a third aspect, the invention relates to a tangible machine readable storage medium comprising instructions, which when read, cause a machine or a device (for instance, a computer unit, a computer, a TPS, or a hardware computing unit) to determine an interest of applying a QA procedure to a treatment plan in radiation therapy by performing the following steps:
- reading information relating to said treatment plan, and information relating to a reference treatment plan that complies with a quality criterion;
- comparing:
   - a dose distribution resulting from said treatment plan with a dose distribution resulting from said reference treatment plan, and/or
   - a parameter of said treatment plan with same parameter of said reference treatment plan;
- determining an interest of applying a QA procedure to said treatment plan from said comparison.

The advantages of the method and of the device of the invention apply to the tangible machine readable storage medium, mutatis mutandis. Different embodiments of this tangible machine readable storage medium are possible. In particular, the different embodiments of the method (first aspect of the invention) apply to it, mutatis mutandis. The corresponding advantages of the different embodiments of the method of the invention also apply to it, mutatis mutandis.

According to a fourth aspect, the invention relates to a program for causing a machine or a device (for instance, a computer unit, a computer, a TPS, or a hardware computing unit) to determine an interest of applying a QA procedure to a treatment plan in radiation therapy and comprising a code for allowing said machine (or device) to perform the following steps:
- reading information relating to said treatment plan, and information relating to a reference treatment plan that complies with a quality criterion;
- comparing:
   - a dose distribution resulting from said treatment plan with a dose distribution resulting from said reference treatment plan, and/or
   - a parameter of said treatment plan with same parameter of said reference treatment plan;
- determining an interest of applying a QA procedure to said treatment plan from said comparison.

The advantages of the method and of the device of the invention apply to the program, mutatis mutandis. Different embodiments of this program are possible. In particular, the different embodiments of the method (first aspect of the invention) apply to it, mutatis mutandis. The corresponding advantages of the different embodiments of the method of the invention also apply to it, mutatis mutandis.

The program according to fourth aspect of the invention is for instance a computer program.

### BRIEF DESCRIPTION OF THE DRAWING

These and further aspects of the invention will be explained in greater detail by way of example and with reference to the accompanying drawings in which:
Fig. 1, Fig. 2, and Fig. 3 are schematic representation of the information used in examples of applications of the method of the invention;
Fig. 4 schematically shows an example of a device of the invention.
Fig. 5a and Fig. 5b show examples of data representing the energy levels of successive layers for a reference treatment plan, and a treatment plan to be evaluated, respectively.
Fig. 6a and Fig. 6b show examples of data representing spot positions and spot weights (i.e. dose) for a reference treatment plan, and for a treatment plan to be evaluated, respectively.
The drawings of the figures are neither drawn to scale nor proportioned. Generally, identical components are denoted by the same reference numerals in the figures.

### DETAILED DESCRIPTION OF PREFERRED EMBODIMENTS OF THE INVENTION

According to a first aspect, the invention relates to a method that can support the decision of: "is a QA needed for a treatment plan 1 or not?" In the current situation, this is up to the medical physicist and his experience to decide if a QA of a treatment plan 1 is needed or not.

Concept of treatment plan in radiation therapy is known by the one skilled in the art. A treatment plan comprises various parameters related to the radiation system that is used. A treatment plan is generally determined by a medical physicist, from the knowledge of the desired dose distribution that is imposed generally by a doctor. When X-ray therapy is used, examples of such parameters are: a Multi Leaf Collimator (MLC) shape or position, a dose rate, beam energy, gantry angle, collimator angle. When particle therapy is used, examples of such parameters are: range, range modulation, shape of compensator, spot size, spot position, spot weight, spot tune, energy of an iso-energetic layer, Source to Axis Distance (SAD), range shifter, ridge filter. A treatment plan is generally determined by a Treatment Planning System (TPS) and/or by a machine design. A treatment plan can refer to one or several radiation beams.

Step i of the method of the invention relates to providing a treatment plan 1. It could be for instance a new treatment plan that has been determined by a practitioner because the patient has moved. This treatment plan 1 does not need to be a QA approved treatment plan. A reference treatment plan 2 also needs to be provided. This reference treatment plan 2 satisfies at least one quality criterion. Preferably, this reference treatment plan 2 is a quality approved treatment plan. A quality approved treatment plan is a treatment plan that has passed a QA procedure, and that therefore complies with at least one quality criterion. An example of QA procedure has been detailed above, when discussing related prior art in the field. This description of an exemplary QA procedure is herewith included by reference. According to another embodiment, the reference treatment plan is not a quality approved treatment plan. It could be for instance a treatment plan that is recognized as being a reference treatment plan by a practitioner because it satisfies at least one quality criterion. For instance, it could be a treatment plan that is known to induce a desired dose in a phantom with acceptable margins or errors. A team of practitioners can have such a reference treatment plan, or a collection of reference treatment plans, notably from previous treatments.

In step v, a comparison is carried out. According to a possible embodiment, a dose distribution resulting from (or induced by) said treatment plan 1 is compared with a dose distribution resulting from (or induced by) said reference treatment plan 2. According to another preferred embodiment, at least one parameter of said treatment plan 1 is compared with corresponding at least one parameter of said reference treatment plan 2. Corresponding means that a parameter of said treatment plan 1 is compared with same parameter of said reference treatment plan 2. So, if said parameter is fluence, the comparison of step v according to this preferred embodiment relates to compare fluence of said treatment plan 1, with fluence of said reference treatment plan 2.

For performing the comparison of step v, different mathematical operations can be implemented for instance. Examples of such mathematical operations are: a difference, an integration of differences over a surface, a convolution. Other mathematical operations are nevertheless possible. Preferably, a deviation Δ is determined from this comparison. For instance, this deviation Δ can be the result of a difference, or of a convolution, or of an integration of differences over a surface. More than one deviation Δ could be determined from the comparison of step v.

From the comparison of step v, an interest of applying a QA procedure to said treatment plan 1 is determined (step v). Preferably, said interest is determined from one or more deviations Δ determined from the comparison of step v, and more preferably, from one or more magnitudes of such deviations Δ.

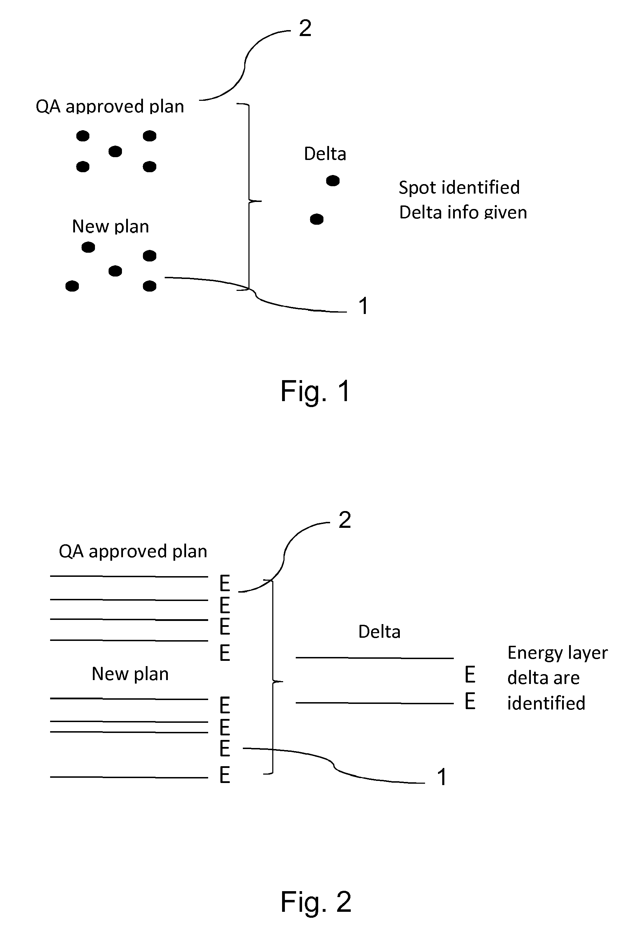

Fig 1, 2, and 3 are schematic representation of the information used in examples of applications of the method of the invention wherein, parameters of a reference treatment plan 2 complying with quality criterion (QA approved plan), of a treatment plan 1 to be assessed (New plan) are shown schematically. The difference between these two sets of perameters (Delta) are shown at the right hand side thereof. These three examples are further discussed below.

Different parameters can be compared in step v between the treatment plan 1 and the quality approved treatment plan 2. One can choose for instance a Source to Axis Distance (SAD). This term is known by the one skilled in the art are represents a distance between an effective source of radiation (for instance a source of protons) and an isocenter. Generally, SAD represents a distance along main beam axis between a radiation source and a rotation axis of the treatment system. In proton therapy, the effective source of protons in a double scattering mode is generally located somewhere between two scatter devices. An exemplary value of SAD is then 222 cm. In proton therapy, the effective source of protons in a wobbling system is generally located somewhere between the two wobbling magnets. An exemplary value of SAD is then 215 cm. For determining SAD in pencil beam scanning particle therapy, one can follow the following method for instance. Spots positions are measured with respect to main beam axis at different depths along said main beam axis. Then, one can evaluate divergence of the beam, and so, the position of the effective source.

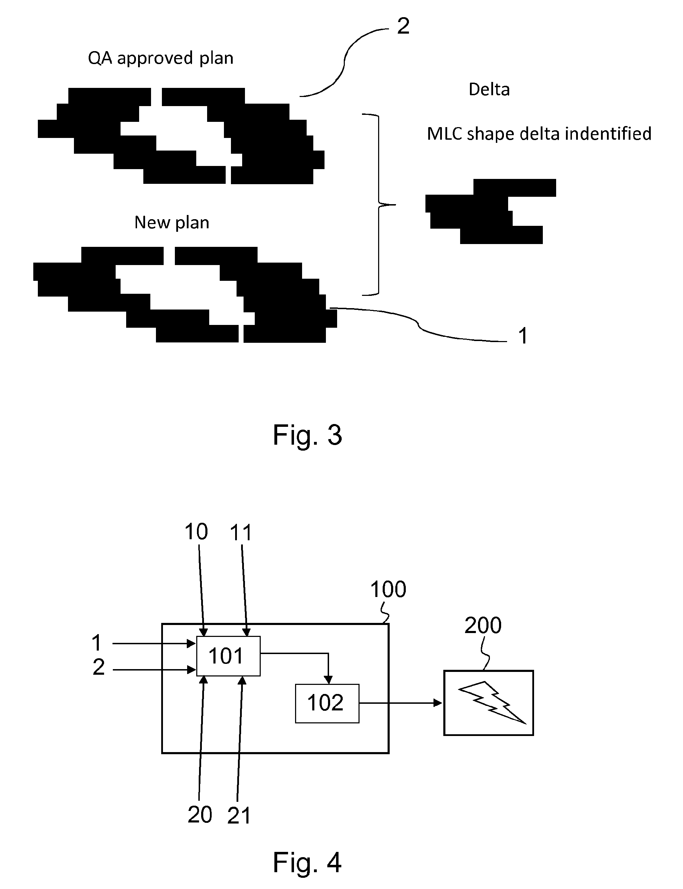

Another example of parameter that can be compared in step v is a Multi Leaf Collimator (MLC) shape. This is an example of a beam limiting device. This is the example shown of Fig. 3. Then, radiation therapy preferably refers to X-ray therapy. MLC shape is known by the one skilled in the art. A set of leafs generally defines the irradiated zone for a given beam and for a given patient. This set is generally placed between the beam source and the patient and is characterized by its shape, the MLC shape.

Another example of parameter that can be compared in step v is a dose rate. This parameter is known by the one skilled in the art and refers to the average dose that is delivered by unit time. In a proton therapy context, average dose rate in a target volume depends on the extracted beam intensity, the volume of the target, the beam spreading technique, the transmission efficiency from accelerator to treatment room and the technique for energy variation. Overall efficiency can approach 40% for scattering systems but might be significantly less. In double scattering, a dose rate of 2 Gy/min in average can be obtained for instance. In wobbling systems, dose rate will depend on the number of paintings that are made for each layer. In order to reduce significantly the dose contribution of one painting for safety reasons, the typical dose rate will be more of the order of 1 Gy/min. Higher dose rates can be obtained in single scattering (5Gy/min).

Another example of parameter that can be compared in step v is beam energy when the irradiating beam has a well defined energy. This is the example of Fig. 2. Range is another example of parameter that can be compared in step v, preferably when radiation therapy refers to particle therapy. Range is known by the one skilled in the art. For instance, range adjustment can be performed either by varying the energy of the irradiating beam from the energy selection system or by degrading the energy of the irradiating beam with absorbers, or by both techniques in tandem. To minimize neutron production and preserve beam quality (minimize emittance growth), it is preferred that energy variation from the energy selection system be used as much as possible. For obtaining small ranges in a patient, energy absorbers in the nozzle can be used.

Range modulation is another example of parameter that can be compared in step v, preferably when radiation therapy refers to particle therapy. Range modulation is known by the one skilled in the art and is generally defined as follows: range modulation (often referenced as m90), or Spread-out Bragg Peak (SOBP) length, is defined as a distance in water between the distal and proximal 90 percent points of a maximum dose value. SOBP length refers to a depth, length along which dose is nearly uniform. In double scattering, maximum range modulation generally depends on the choice of specific options. The options providing large fields (typical 22-cm diameter) generally have modulators designed for full modulation. Range modulator for wobbling is generally designed for full modulation. For single scattering, range modulation is generally fixed to a maximum value of 5 cm.

Another example of parameter that can be compared in step v is gantry angle. This term is known by the one skilled in the art. Gantry angle is generally equal to 0° when the associated nozzle is at 12 o'clock. Positive angles are generally clockwise.

Another example of parameter that can be compared in step v is collimator angle that is known by the one skilled in the art. A group of radiation attenuating material with one or more apertures generally defines a field of view and limits the angular spread of the radiating beam. Collimator angle characterizes this limit of angular spread.

Another example of parameter that can be compared in step v is Monitor Unit (MU) that is known by the one skilled in the art. MU is a measure of output of a beam delivery system.

Another example of parameter that can be compared in step v is a set of beam modifying devices, such as wedges, trays for instance. Beam modifying devices are devices generally located in the nozzle and or in the Energy Selection System (ESS). They are used to define or modify an irradiating beam. When located in the ESS, they alter the beam energy and define the beam emittance. When located in the Nozzle they can generally be set and/or positioned differently for a particular irradiation. Examples of beam modifying devices are a range modulator, an additional first scatterer, a second scatterer, scanning/wobbling magnets, a variable collimator, a patient-specific aperture, a range compensator supported by a snout.

Another example of parameter that can be compared in step v is a shape of blocks that is known by the one skilled in the art. Then radiation therapy preferably refers to particle therapy. To be able to avoid irradiation of normal tissue, one can use one or more blocks that can stop a radiation beam or that can highly reduce its intensity in some areas.

Another example of parameter that can be compared in step v is a shape of range compensator. Then radiation therapy preferably refers to particle therapy. Shape of range compensator is known by the one skilled in the art. It generally refers to a bloc of range shifting material, shaped on one face (normally the upstream face) in such a way that the distal end of a particle field in the patient takes the shape of the distal end of the target volume. A range compensator is generally supported by and positioned in the snout, and is normally located just downstream of the aperture.

Another example of parameter that can be compared in step v is a spot size. Then radiation therapy preferably refers to particle therapy using a Pencil Beam Scanning (PBS) technique. As it is known by the one skilled in the art, when using a pencil beam scanning technique, different layers are generally defined along and generally perpendicular to main axis of the radiating beam. These different layers are therefore positioned at different depths. In the different layers, spots are defined for irradiating a target area. Spot size is known by the one in the art and refers to the size of a spot. Spot size, is generally of the order of 3 to 6 mm, one sigma, depending of beam energy.

Another example of parameter that can be compared in step v is spot position, or the positions of the different spots. This is the example of Fig. 1. Then radiation therapy preferably refers to particle therapy using a PBS technique. In each layer used in pencil beam scanning technique, different spots are generally defined having different coordinates x, y, or different positions, for irradiating a target area.

Another example of parameter that can be compared in step v is spot weight, and preferably relative spot weight. Then radiation therapy preferably refers to particle therapy using a PBS technique. Spot weight relates to the intensity related to a spot. Relative spot weight means that an intensity normalized to a reference intensity is considered rather than an absolute intensity.

Another example of parameter that can be compared in step v is spot tune. Then radiation therapy preferably refers to particle therapy using a PBS technique. Spot tune is known by the one skilled in the art and characterizes width and divergence of an elementary pencil beam at isocenter in pencil beam scanning. Another example of parameter that can be compared in step v is a number of spot, preferably per layer.

Another example of parameter that can be compared in step v is a number of layers when PBS technique is used in particle therapy. Still another example of parameter that can be compared in step v is an energy of a layer or the energies of the different layers. Still another example of parameter that can be compared in step v is a weight of a layer. When repainting is used (repainting is known by the one skilled in the art), other examples for the parameter that can be compared in step v are: a number of painting, a kind of painting, a time frame specified for the repainting. Rather than delivering the whole desired dose once, it is often preferred to deliver the dose with successive iterations, by 'repainting' the different layers, ie by irradiating the different spots several times successively. This allows increasing safety of the treatment.

Another example of parameter that can be compared in step v is range shifter, or a (or more) setting or defining parameter of a range shifter. Then radiation therapy preferably refers to particle therapy, preferably using a PBS technique. Range shifter is known by the one skilled in the art and allows modifying range in a target volume (a patient for instance).

Another example of parameter that can be compared in step v is ridge filter, or a (or more) setting or defining parameter of a ridge filter. Ridge filter is known by the one skilled in the art and refers to an accessory used to spread out the depth of Bragg peaks. A ridge filter is particularly useful at low range in PBS, where Bragg peaks are very narrow.

Other examples of the parameter that can be compared in step v are possible. For instance, this parameter could be: patient positioner position that generally comprises the following set of coordinates: (X, Y, Z, pitch, roll, rot). A subset of (X, Y, Z, pitch, roll, rot) could also be used for the parameter of step v.

Preferably, several parameters are compared in step v. More preferably, the following four parameters are compared in step v: energy, spot position(s), spot weight(s), spot size(s). Said energy can be for instance a nominal beam energy, when a photon beam is used. When pencil beam scanning (PBS) technique is used in particle therapy, said energy preferably refers to an energy of the used particles or to an energy of a layer. More preferably, different energies could be compared in step v, possibly with other parameters, for instance with three other parameters such as spot position(s), spot weight(s), spot size(s). Such different energies could refer to the energies of different layers.

Comparison of step v comprises for instance a step of determining a difference between a dose distribution induced by said treatment plan 1 and another induced by said reference treatment plan 2. Then, it is for instance possible to calculate such a difference on a point to point basis if a target volume has been discretized. For each point of the target volume, one has then local deviations Δᵢ, where subscript i refers to a point in the target volume. From these different local deviations Δᵢ, it is possible to determine a mean deviation Δ or to integrate the different Δᵢ over the target volume for obtaining a global deviation Δ. Then, depending on the magnitude of said mean or global deviation Δ, it is possible to determine the interest of applying a QA procedure to the treatment plan 1 (see before for instance). According to another possible embodiment, the different local deviations Δᵢ, are used for determining said interest of applying a QA procedure to the treatment plan 1. For instance, it can be decided that there is no interest in applying a QA procedure to the treatment plan 1 if no local deviations Δᵢ is larger than a given threshold, or if the number of local deviations Δᵢ larger than a given threshold is smaller than a given number. Comparison of step v between a dose distribution resulting from said treatment plan 1 and said reference treatment plan 2 can also comprise a step of determining these two dose distributions that have been integrated over a given surface, or over a given volume. Thereafter, one could for instance make a difference between such two integrated dose distributions for determining a deviation Δ that would be used in step vi for determining an interest in applying a QA procedure to said treatment plan 1.

Preferably, several parameters are compared in step v. To perform such a comparison, several procedures are also possible. If four parameters are compared in step v, one could choose for instance the following procedure: determining four intermediate deviations, Δⱼ, where subscript j=1, ..., 4 relates to each of said four parameters. One intermediate deviation, Δⱼ, characterizes a difference between one parameter of said treatment plan 1 and same parameter of said reference treatment plan 2. If said parameter is 'spot position', Δⱼ, could designate, for instance, a mean difference between spot positions between said treatment plan 1 and said reference treatment plan 2. If said parameter is a beam energy, Δⱼ, could designate, for instance, a difference between a beam energy of said treatment plan 1 and a beam energy of said reference treatment plan 2. From these intermediate deviations, Δⱼ, one can then, according to a possible embodiment, determine a global deviation Δ. Depending on its magnitude, interest of applying a QA procedure to the treatment plan 1 can then be determined. According to another possible embodiment, these different intermediate deviations, Δⱼ, are directly used for determining if there is an interest of applying such a QA procedure. For instance, different thresholds Δ₂ⱼ could be pre-determined by a practitioner for the different parameters. And, interest of applying a QA procedure to the treatment plan 1 would be high only if each intermediate deviation, Δⱼ, is larger than the corresponding threshold, Δ₂ⱼ. Another possibility is to determine different lower threshold Δ₁ⱼ for the different parameters that are compared in step v, and to determine that a QA procedure does not need to be applied if each intermediate deviation, Δⱼ, is lower than the corresponding lower threshold, Δ₁ⱼ. According to another possible embodiment, it could be determined that a QA procedure does not need to be applied if at least half of the intermediate deviations, Δⱼ, are lower than their corresponding lower threshold, Δ₁ⱼ.

An example of scalar parameter that can be compared in step v is a coordinate of a spot position, for instance its X coordinate. Then, an exemplary value for Δ₂ is 1 cm.

According to a second aspect, the invention relates to a device 100 for determining an interest of applying a QA procedure to a treatment plan 1. An example of such a device 100 is schematically shown in Fig.4, in combination with a display 200. Examples of said device 100 are: a computer, a TPS, a hardware unit of a TPS, a hardware unit able to communicate with a TPS. The device 100 comprises input means 101 for inputting or receiving information relating to a treatment plan 1, and information relating to a reference treatment plan 2 that complies with a quality criterion. In some embodiments, the input means 101, may also be used for receiving a dose distribution 20 resulting from said reference treatment plan 2 and/or a parameter 21 of said reference treatment plan 2 and/or a dose distribution 10 resulting from said treatment plan 1 and/or a parameter 11 of said treatment plan 1.
Examples of input means 101 are: an input port of the device 100, for instance a USB port, an Ethernet port, a wireless (Wi-Fi for instance) input port. Other examples of input means 101 are nevertheless possible. The device 100 further comprises processing means 102 for comparing:
▪ a dose distribution resulting from said treatment plan 1 with a dose distribution resulting from said reference treatment plan 2, and/or
▪ a parameter of said treatment plan 1 with same parameter of said reference treatment plan 2.
Processing means 102 is also able to determine an interest of applying a QA procedure to said treatment plan 1 from said comparison, for instance from a deviation Δ between said treatment plan 1 and said reference treatment plan 2 resulting from this comparison step. In some embodiments of the invention, the processing means 102 may be used for computing a dose distribution 20 resulting from said reference treatment plan 2 and/or a parameter 21 of said reference treatment plan 2 and/or a dose distribution 10 resulting from said treatment plan 1 and/or a parameter 11 of said treatment plan 1.

Examples of said processing means 102 are a computing unit, a central processing unit (CPU), a controller, a ship, a microchip, an integrated circuit (IC), a multi-core processor, a system on chip (SoC), a control unit, an array processor, or another type of processor known by the one skilled in the art. According to a possible embodiment, said processing means 102 comprises different units for the steps of comparing, and determining the interest of applying a QA procedure to the treatment plan 1. As shown in figure 4, the device 100 is preferably able to send information to a display 200. For instance, the device 100 sends to said display 200 said interest of applying a QA procedure to said treatment plan 1. Said interest is for instance displayed in a form of a flag that can take several colors (see above), depending of the level of said interest.

According to a third aspect, the invention relates to a tangible machine readable storage medium comprising instructions, which when read, cause a machine 100 to determine an interest of applying a QA procedure to a treatment plan 1 in radiation therapy by performing the following steps:
- reading information relating to said treatment plan 1, and information relating to a reference treatment plan 2 that complies with a quality criterion;
- comparing:
   - a dose distribution resulting from said treatment plan 1 with a dose distribution resulting from said reference treatment plan 2, and/or
   - a parameter of said treatment plan 1 with same parameter of said reference treatment plan 2;
- determining an interest of applying a QA procedure to said treatment plan 1 from said comparison.

According to a fourth aspect, the invention relates to a program For causing a machine 100 to determine an interest of applying a QA procedure to a treatment plan 1 in radiation therapy and comprising a code for allowing said machine 100 to perform the following steps:
- reading information relating to said treatment plan 1, and information relating to a reference treatment plan 2 that complies with a quality criterion;
- comparing:
   - a dose distribution resulting from said treatment plan 1 with a dose distribution resulting from said reference treatment plan 2, and/or
   - a parameter of said treatment plan 1 with same parameter of said reference treatment plan 2;
determining an interest of applying a QA procedure to said treatment plan 1 from said comparison.

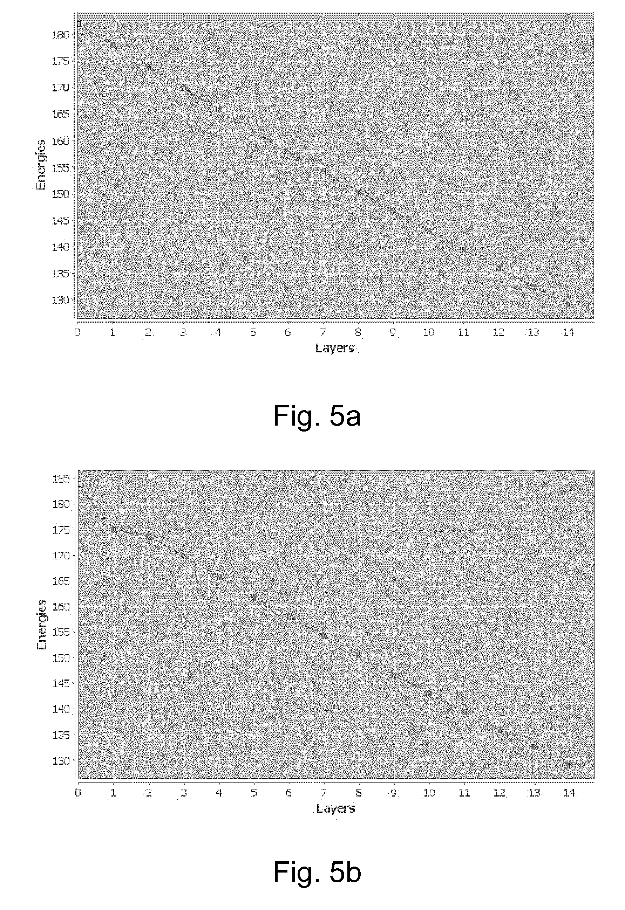

Fig. 5a and Fig. 5b show another example of data representing the energy levels of successive layers for a reference treatment plan, and a treatment plan to be evaluated. Both the reference treatment plan of Fig. 5a and the treatment plan to be evaluated of Fig. 5b comprise 15 energy levels corresponding to 15 layers to be irradiated. In the reference treatment plan of Fig. 5a, layer #0 has a nominal beam energy of 182.06 MeV and layer #1 has a nominal beam energy of 177,97 MeV. In the treatment plan to be evaluated of Fig. 5b, layer #0 has a nominal beam energy of 184 MeV and layer #1 has a nominal beam energy of 175 MeV. All other beam energies and all other parameters of the treatment plans are identical. A comparison of both treatment plans is performed and reveals the two differences and the magnitude of the deviation. The comparison may be performed using the DICOM data of both treatment plans. Alternatively, dose distributions may be obtained or computed for both treatment plans, and the dose distributions may be compared and a difference of the dose distributions may be computed. The interest of applying a QA procedure to the treatment plan of Fig. 5b will be determined by this comparison. The criteria that will trigger the interest will be configurable parameters.

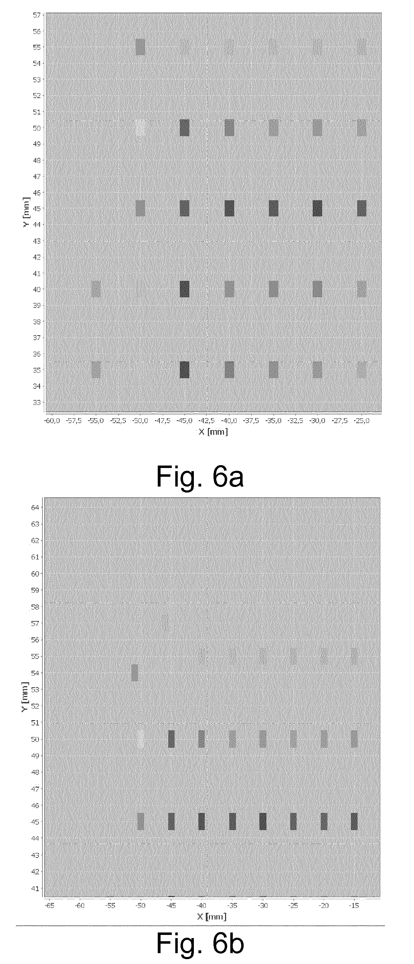

Fig. 6a and Fig. 6b show another example of data representing spot positions for a reference treatment plan, and for a treatment plan to be evaluated. Fig. 6a represents the X and Y positions of a set of 32 spots to be irradiated in a layer of a reference treatment plan. The different grey levels correspond to different spot weights (i.e spot doses). Fig 6b shows the upper three lines of spots for a treatment plan to be evaluated. A comparison is performed between the parameters of the two treatment plans and reveals that only two spots have been shifted, the spot at (-50mm, 55mm) was shifted to (-50mm, 54mm) and the spot at (-45mm, 55mm) was shifted to (-45mm, 57mm), and that all other parameters were unchanged. The comparison may be performed using the DICOM data of both treatment plans. Alternatively, dose distributions may be obtained or computed for both treatment plans, and the dose distributions may be compared and a difference of the dose distributions may be computed. From the evaluation of the importance of these differences, a decision can be made as to the necessity of performing a QA procedure to the treatment plan of Fig. 6b. The decision may take the magnitude of the difference into account and compare this magnitude to a threshold. The magnitude of the difference may be a weighted combination of the differences of various parameters, such as Multi Leaf Collimator (MLC) shape, dose rate, beam energy, gantry angle, collimator angle, range, range modulation, shape of compensator, spot size, spot position, spot weight, spot tune, energy of an iso-energetic layer, Source to Axis Distance (SAD), range shifter, ridge filter, or other parameters of the treatment plan.

The present invention has been described in terms of specific embodiments, which are illustrative of the invention and not to be construed as limiting. More generally, it will be appreciated by persons skilled in the art that the present invention is not limited by what has been particularly shown and/or described hereinabove. Reference numerals in the claims do not limit their protective scope. Use of the verbs "to comprise", "to include", or any other variant, as well as their respective conjugations, does not exclude the presence of elements other than those stated. Use of the article "a", "an" or "the" preceding an element does not exclude the presence of a plurality of such elements.

The invention can also be summarized as follows. The method of the invention comprises the steps of: providing information relating to a treatment plan 1; providing information relating to a reference treatment plan 2; comparing a dose distribution resulting from said treatment plan 1 with a dose distribution resulting from said reference treatment plan 2, and/or a parameter of said treatment plan 1 with same parameter of said reference treatment plan 2; from said comparison, determining an interest of applying a QA procedure to said treatment plan 1.

## Claims

1. Computer-implemented method for determining an interest of applying a Quality Assessment (QA) procedure to a treatment plan (1) in radiation therapy and comprising the steps of:
i. receiving information relating to said treatment plan (1);
ii. receiving information relating to a reference treatment plan (2) that complies with a quality criterion,
iii. obtaining a dose distribution (20) resulting from said reference treatment plan (2) and/or a parameter (21) of said reference treatment plan (2);
iv. obtaining a dose distribution (10) resulting from said treatment plan (1) and/or a parameter (11) of said treatment plan (1);
v. comparing:
• said dose distribution resulting from said treatment plan (10) with said dose distribution resulting from said reference treatment plan (20), and/or
• said parameter of said treatment plan (11) with same parameter of said reference treatment plan (21);
vi. from said comparison of step v, determining an interest of applying a QA procedure to said treatment plan (1).

2. Method according to claim 1 **characterized in that** said dose distribution (10) resulting from said treatment plan (1) and/or said parameter (11) of said treatment plan (1) is obtained from a treatment planning system.

3. Method according to claim 1 **characterized in that** said dose distribution (10) resulting from said treatment plan (1) and/or said parameter (11) of said treatment plan (1) is obtained by a computation.

4. Method according to any of preceding claims **characterized in that** said dose distribution (20) resulting from said reference treatment plan (2) and/or said parameter (21) of said reference treatment plan (2) is obtained from a treatment planning system.

5. Method according to any of preceding claims **characterized in that** said dose distribution (20) resulting from said reference treatment plan (2) and/or said parameter (21) of said reference treatment plan (2) is obtained by a computation.

6. Method according to any of previous claims **characterized in that** the method is performed prior to the delivery of said treatment plan (1).

7. Method according to any of preceding claims **characterized in that** said comparison in step v comprises a step of determining a deviation Δ between said treatment plan (1) and said reference treatment plan (2).

8. Method according to any of previous claims **characterized in that** said step v comprises a step of determining that a QA procedure should be applied to said treatment plan (1) if magnitude of said deviation Δ is larger than a threshold, Δ₂.

9. Method according to claim 7 or 8 **characterized in that** said step v comprises a step of determining that a QA procedure does not need to be applied to said treatment plan (1) if magnitude of said deviation Δ is lower than a threshold, Δ₁.

10. Method according to any of claims 7 to 9 **characterized in that** said parameter (11, 21) that is compared between said treatment plan (1) and said reference treatment plan (2) in step v is able to influence a fluence in radiation therapy.

11. Method according to any of previous claims **characterized in that** said parameter (11, 21) that is compared between said treatment plan (1) and said reference treatment plan (2) in step v is a fluence.

12. Method according to any of previous claims **characterized in that** said parameter (11, 21) that is compared between said treatment plan (1) and said reference treatment plan (2) in step v is a beam energy.

13. Method according to any of previous claims **characterized in that** said parameter (11, 21) that is compared between said treatment plan (1) and said reference treatment plan (2) in step v is a spot size.

14. Method according to any of previous claims **characterized in that** said parameter (11, 21) that is compared between said treatment plan (1) and said reference treatment plan (2) in step v is a spot position.

15. Method according to any of previous claims **characterized in that** said parameter (11, 21) that is compared between said treatment plan (1) and said reference treatment plan (2) in step v is a spot weight.

16. Method according to any of previous claims **characterized in that** said parameter (11, 21) that is compared between said treatment plan (1) and said reference treatment plan (2) in step v is a beam limiting device position.

17. Method according to any of previous claims **characterized in that** said step v comprises a step of comparing the following four parameters between said treatment plan (1) and said reference treatment plan (2): energy, spot position, spot weight, and spot size.

18. Device (100) for determining an interest of applying a Quality Assessment (QA) procedure to a treatment plan (1) in radiation therapy and comprising:
- input means (101) for receiving:
▪ information relating to said treatment plan (1),
▪ information relating to a reference treatment plan (2) that complies with a quality criterion,
- input means (101) for receiving and/or processing means (102) for computing:
▪ a dose distribution (20) resulting from said reference treatment plan (2) and/or a parameter (21) of said reference treatment plan (2), and/or
▪ a dose distribution (10) resulting from said treatment plan (1) and/or a parameter (11) of said treatment plan (1);
- processing means (102) for:
▪ comparing:
• said dose distribution (10) resulting from said treatment plan (1) with said dose distribution (20) resulting from said reference treatment plan (2), and/or
• said parameter (11) of said treatment plan (1) with same parameter (21) of said reference treatment plan (2); and for
▪ determining an interest of applying a QA procedure to said treatment plan (1) from said comparison.

19. Device (100) according to claim 18 **characterized in that** said processing means (102) are able to determine a deviation Δ between said treatment plan (1) and said reference treatment plan (2) from said comparison.

20. A tangible machine readable storage medium comprising instructions, which when read, cause a machine (100) to determine an interest of applying a Quality Assessment (QA) procedure to a treatment plan (1) in radiation therapy by performing the following steps:
- reading information relating to said treatment plan (1), and information relating to a reference treatment plan (2) that complies with a quality criterion;
- comparing:
• a dose distribution resulting from said treatment plan (1) with a dose distribution resulting from said reference treatment plan (2), and/or
• a parameter of said treatment plan (1) with same parameter of said reference treatment plan (2);
- determining an interest of applying a QA procedure to said treatment plan (1) from said comparison.

21. Program for causing a machine (100) to determine an interest of applying a Quality Assessment (QA) procedure to a treatment plan (1) in radiation therapy and comprising a code for allowing said machine (100) to perform the following steps:
- reading information relating to said treatment plan (1), and information relating to a reference treatment plan (2) that complies with a quality criterion;
- comparing:
• a dose distribution resulting from said treatment plan (1) with a dose distribution resulting from said reference treatment plan (2), and/or
• a parameter of said treatment plan (1) with same parameter of said reference treatment plan (2);
- determining an interest of applying a QA procedure to said treatment plan (1) from said comparison.
